Europäisches Patentamt

(19) European Patent Office

Office européen des brevets

(11) Publication number: **0 257 802**
**B1**

(12) **EUROPEAN PATENT SPECIFICATION**

(45) Date of publication of patent specification: **27.12.90**

(21) Application number: **87306582.5**

(22) Date of filing: **24.07.87**

(51) Int. Cl.⁵: **A 61 F 5/448**

(54) Ostomy coupling.

(30) Priority: **31.07.86 GB 8618692**
**13.08.86 GB 8619715**

(43) Date of publication of application:
**02.03.88 Bulletin 88/09**

(45) Publication of the grant of the patent:
**27.12.90 Bulletin 90/52**

(84) Designated Contracting States:
**AT BE CH DE ES FR GR IT LI LU NL SE**

(56) References cited:
**EP-A-0 068 778**
**EP-A-0 098 718**
**GB-A-1 568 860**
**GB-A-2 115 288**
**GB-A-2 121 902**
**US-A-4 460 363**

(73) Proprietor: **E.R. Squibb & Sons, Inc.**
**Lawrenceville-Princeton Road**
**Princeton, N.J. 08540-4000 (US)**

(72) Inventor: **Steer,Peter Leslie**
**"Kingscote",Woodlands Rise**
**East Grinstead,Sussex (GB)**
Inventor: **Wiltshire,Neil Phillip**
**1 Ormus Cottages Newchapel Road**
**Lingfiled Surrey (GB)**

(74) Representative: **Cook, Anthony John et al**
**D. YOUNG & CO. 10, Staple Inn**
**London, WC1V 7RD (GB)**

Courier Press, Leamington Spa, England.

## Description

The present invention relates to a coupling for releasably connecting a bag or pouch (for receiving discharged waste) to a pad attached to the body of a wearer. Such bags are often called "ostomy bags" and such couplings are often called "ostomy couplings".

It is an aim of the present invention to provide a coupling which is simple and economical to manufacture, comprises a minimum number of parts, and provides a secure and leak-proof attachment. One successful prior design of two part ostomy coupling is shown in U.S. Patent No. 4,460,363 entitled OSTOMY BAG which issued to P. L. Steer, et al on July 17th 1984. Another design, which is preferable from the points of view of flexibility and reduced thickness is shown in British Patent No. 2 121 902. Reduced overall thickness is desirable, so that the appliance, when worn under light or sports clothing, is less obtrusive. It is also desirable that an ostomy coupling can be separated using a relatively small uncoupling force, without adversely affecting its leakproof quality.

According to the invention, there is provided an ostomy coupling having two interengaging coupling elements one of which carries a seal and the other of which has a cooperating sealing surface, the body-side coupling element having a chute encircled by an array of arcuate walls, the walls having inwardly projecting portions which are positioned to be capable of overlapping an outwardly projecting portion of a circular member on the said one coupling element, said arcuate walls being spaced radially outwardly of the chute, characterised in that each of the arcuate walls is substantially coextensive with, and adjacent and concentric with an arcuate slot extending through an annular flange of the body-side coupling element. These slots are provided so that the body-side coupling can be made in a single injection molding operation despite the fact that it has a re-entrant structure when seen in radial cross-section.

Both of the coupling elements are made of a slightly resilient flexible plastic, and the extent of overlap in the radial direction is chosen so that the coupling elements can be manually separated. No relative rotation is needed.

An advantage of this coupling is that by choosing varying arcuate lengths for the arcuate walls, the force required to uncouple the coupling can be varied, without any effect on the quality of the coupling regarding its liquid sealing capabilities. The basic design is thus versatile in the sense that an ostomy coupling may be designed for any desired uncoupling force.

Also, according to the invention there is provided a method of manufacturing an ostomy coupling element which comprises a radially-extending substantially planar flange and a substantially cylindrical wall which surrounds a stomal orifice in the flange, there being an array of projections encircling the wall, each of the projections having a radially inwardly-extending portion, the method comprising injection molding the element in one piece from a synthetic plastics material in such a manner as to define the inwardly extending portions by respective tools extending axially through the flange, and then removing the element from the mold in an axial direction away from the said tools.

Further, according to the invention there is provided a body-side ostomy coupling element comprising a substantially planar flange having a stomal orifice surrounded by a substantially cylindrical wall, an array of arcuate walls encircling the cylindrical wall, there being a radially inwardly projecting portion of at least some of the arcuate walls, characterised by an arcuate slot through the flange adjacent to each arcuate wall that has the aforesaid projection, the slots being in a circular array located radially inwardly of the arcuate walls.

In the drawing:

Fig. 1 illustrates, in axial cross-section, one example of bag-side and body-side coupling elements in uncoupled condition, and

Fig. 2 illustrates these elements in coupled condition.

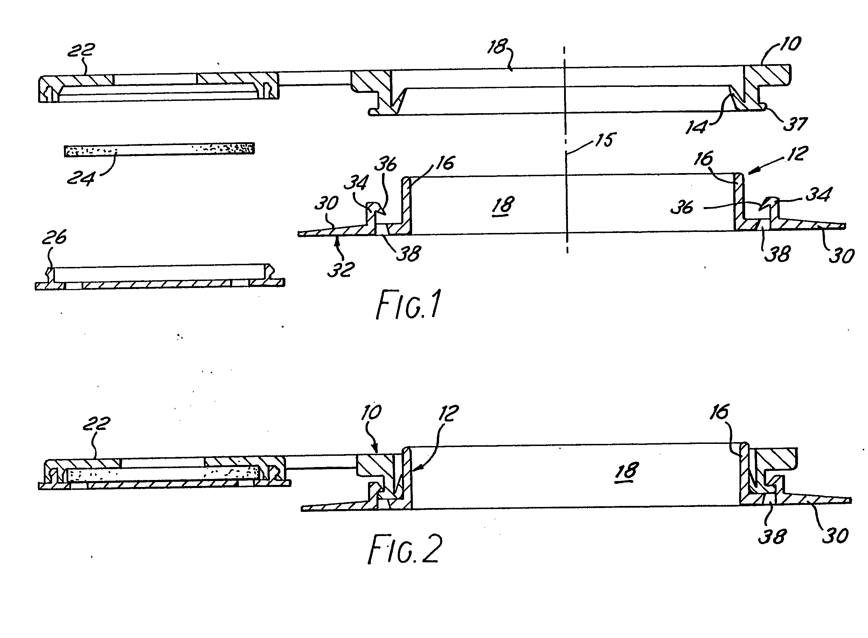

The illustrated ostomy coupling includes two inter-engaging coupling elements, a bag-side coupling element 10 and a body-side coupling element 12. As shown in Fig. 1, the bag-side coupling element 10 has a peripheral deflectable seal 14 thereon, extending radially inwardly. The line 15 indicates the common axis of the couplings 10, 12 around a stomal orifice 18. The deflectable seal 14 cooperates with the external wall of a chute 16 on the body-side coupling element 12. Such a seal is more fully described and claimed in British Patent No. 1568860 to which the reader is referred. As illustrated in the Figures, the bag-side coupling element 10 includes a filter housing 22 which can contain filter media 24 and may have a cover or lid 26. For a fuller description of these parts, reference should be made to British Patent Application 8531257 (EP—A—210032). However, the filter housing 22 is not an essential part of the present invention and can be omitted if desired.

The body-side coupling element 12, comprises a flange 30 whose surface 32 is intended for attachment in any suitable way to a pad of medical or surgical adhesive. Made in one piece with the flange 30 is a cylindrical wall 16 constituting a chute for guiding waste material to the interior of a bag or pouch. It will be observed that when the coupling elements 10, 12 are assembled as shown in Fig. 2, the wall 16 prevents any fecal waste material discharged by the wearer and conducted by the chute to the bag interior from having access to, or being able to lodge in, crevices in the other part 10 of the coupling.

Also made in one piece with, and forming part of, the body-side coupling element 12 are a series of arcuate walls 34 arranged to constitute an interrupted encircling wall. Each of the walls 34 has an inwardly projecting flange 36. As seen, the flanges 36 taper towards their radially inward end, this construction being adopted to make them flexible and capable of deformation. In assembling the two coupling elements 10, 12 together, the elements 10, 12 are moved axially towards each other and the rim 36 on the element 10 is forced past the flanges 36. Due to their inherent resilience, these flanges then spring outwardly once the coupling elements are in their relative position illustrated in Fig. 2.

It will be realized from an inspection of the Figures that the presence of flanges 36 leads to a reentrant structure which would preclude the molding of the body-side coupling element 12 as a single part. In the region of each arcuate wall 34, the flange 30 has an arcuate slot 38 whose purpose is to permit the passage of a suitable molding tool so that the form of the inwardly directed flange 36 can be properly determined. With this slotted construction, it is possible to produce the body-side coupling element in a single injection molding operation with consequent economy and efficiency of manufacture.

In one embodiment of the invention, there may be eight arcuate walls 34, each subtending an angle at the axis of 45 degrees, and each one spaced from its neighbour by a similar angle. In an alternative embodiment, there may be six arcuate walls 34, each subtending an angle of 30 degrees at the axis.

The force required to separate the two coupling parts can be adjusted by adjusting the inward overlap of the flange 36 relative to the rim 37, and an alternative method of adjusting this force is to vary the arcuate extent of the walls 34. Because of the engagement of the seal 14 with the chute wall 16, variations can be made without affecting the liquid sealing capabilities of the coupling.

**Claims**

1. An ostomy coupling having two interengaging coupling elements one (10) of which carries a seal and the other (12) of which has a cooperating sealing surface, the body-side coupling element (12) having a chute (16) encircled by an array of arcuate walls (34), the walls having inwardly projecting portions (36) which are positioned to be capable of overlapping an outwardly projecting portion (37) of a circular member on the bag-side coupling element (10), said arcuate walls (34) being spaced radially outwardly of the chute (16), characterised in that each of the arcuate walls (34) is substantially coextensive with, and adjacent and concentric with an arcuate slot (38) extending through an annular flange (30) of the body-side coupling element (12).

2. A method of manufacturing an ostomy coupling element (12) which comprises a radially-extending substantially planar flange (30) and a substantially cylindrical wall (16) which surrounds a stomal orifice (18) in the flange (30), there being an array of projections (34) encircling the wall (16) each of the projections having a radially inwardly-extending portion (36), the method comprising injection molding the element in one piece from a synthetic plastics material in such a manner as to define the inwardly-extending portions by respective tools extending axially through the flange, and then removing the element (12) from the mold in an axial direction away from the said tools.

3. A body-side ostomy coupling element (12) comprising a substantially planar flange (30) having a stomal orifice (18) surrounded by a substantially cylindrical wall (16), an array of arcuate walls (34) encircling the cylindrical wall (16), there being a radially inwardly projecting portion (36) of at least some of the arcuate walls (34), characterised by an arcuate slot (38) through the flange (30) adjacent to each arcuate wall that has the aforesaid projection, the slots (38) being in a circular array located on radially inwardly of the arcuate walls (34).

**Patentansprüche**

1. Ostomiekupplung mit zwei miteinander in Eingriff bringbaren Kupplungselementen, wobei eines (10) eine Dichtung trägt und das andere (12) eine zusammenwirkende Dichtungsfläche hat, wobei das körperseitige Kupplungslement (12) eine Rutsche (16) aufweist, die von einer Reihe gebogener Wände (34) umgeben ist, die Wände nach innen vorspringende Abschnitte (36) aufweisen, die so angeordnet sind, daß sie mit einem nach außen vorspringenden Abschnitt (37) eines kreisförmigen Elements an dem taschenseitigen Kupplungselement (10) überlappen können, die gebogenen Wände (34) radial außerhalb der Rutsche (16) beabstandet angeordnet sind, dadurch gekennzeichnet, daß jede der gebogenen Wände (34) im wesentlichen in Verlängerung zu, und benachbart und konzentrisch zu einem gebogenen Schlitz (38) angeordnet ist, der sich durch einen ringförmigen Flansch (30) des körperseitigen Kupplungselements (12) erstreckt.

2. Verfahren zum Herstellen eines Ostomiekupplungselements (12) das einen sich radial nach außen erstreckenden, im wesentlichen flachen Flansch (30) aufweist und eine im wesentlichen zylindrische Wand (16), die eine Stomaöffnung (18) in dem Flansch (30) umgibt, wobei eine Reihe von Vorsprüngen (34), die die Wand (16) umgeben, vorhanden sind, wobei jeder der Vorsprünge einen radial nach innen sich erstreckenden Abschnitt (36) aufweist, wobei das Verfahren umfaßt, Spritzgießen des Elements in einem Stück aus einem synthetischen Kunststoffmaterial in solcher Weise, so daß die sich nach innen erstreckenden Abschnitte von entsprechenden Werkzeugen, die sich axial durch den Flansch erstrecken, definiert werden und dann Herausnehmen des Element (12) in einer axialen Richtung weg von den Werkzeugen aus der Form.

3. Körperseitiges Ostomiekupplungselement (12) mit einem im wesentlichen ebenen Flansch (30), der eine Stomaöffnung (18) aufweist, die von einer im wesentlichen zylindrischen Wand (16) umgeben ist, einer Reihe von gebogenen Wänden (34), die die zylindrische Wand (16) umgeben, wobei ein radial nach innen vorspringender Abschnitt (36) von mindestens einigen der gebogenen Wände (34) vorhanden ist, gekennzeichnet durch einen bogenförmigen Schlitz (38) durch den Flansch (30) benachbart zu jeder gebogenen Wand, die den zuvor genannten Vorsprung hat, wobei die Schlitze (38) in einem kreisförmigen Feld radial innerhalb der gebogenen Wände (34) angeordnet sind.

## Revendications

1. Raccord de stomie comportant deux éléments de raccord à prise mutuelle, dont l'un (10) porte un joint d'étanchéité et l'autre (12) a une surface d'étanchéité complémentaire, l'élément de raccord côté corps (12) comportant une goulotte (16) encerclée par une série de parois (34) en arc-de-cercle, ces parois comportant des parties (36) en saillie vers l'intérieur qui sont disposées de façon à pouvoir recouvrir partiellement une partie (37), en saillie vers l'extérieur, d'une pièce circulaire sur l'élément de raccord côté poche (10), lesdites parois (34) en arc-de-cercle étant espacées radialement vers l'extérieur de la goulotte (16), caractérisé en ce que chacune des parois (34) en arc-de-cercle a sensiblement la même étendue que, et est adjacente et concentrique à une fente (38) en arc-de-cercle qui traverse une bride annulaire (30) de l'élément de raccord côté corps (12).

2. Procédé de fabrication d'un élément de raccord de stomie (12) qui comprend une bride radiale sensiblement plane (30) et une paroi sensiblement cylindrique (16) entourant un orifice de stomie (18) pratiqué dans la bride (30), une série de saillies (34) encerclant la paroi (16), chacune des saillies comportant une partie (36) dirigée radialement vers l'intérieur, le procédé consistant à mouler par injection l'élément en une seule pièce à partir d'une matière plastique synthétique de manière à faire forme les parties dirigées vers l'intérieur par des outils respectifs traversant axialement la bride, puis à retirer l'élément (12) du moule dans une direction axiale opposée auxdits outils.

3. Elément de raccord de stomie côté corps (12) comprenant une bride sensiblement plane (30) qui comporte un orifice de stomie (18) entouré par une paroi sensiblement cylindrique (16) une série de parois (34) en arc-de-cercle encerclant la paroi cylindrique (16), au moins certaines des parois (34) en arc-de-cercle comportant une partie (36) en saillie radialement vers l'intérieur, caractérisé par une fente (38) en arc-de-cercle traversant la bride (30) à proximité de chaque paroi en arc-de-cercle qui comporte la saillie précitée, les fentes (38) formant une série circulaire située radialement à l'intérieur des parois (34) en arc-de-cercle.

FIG.1

FIG.2

EP 0 257 802 B1